# EUROPÄISCHE PATENTSCHRIFT

(11) **EP 1 988 863 B1**
(45) Veröffentlichungstag und Bekanntmachung des Hinweises auf die Patenterteilung: **30.11.2011**
(21) Anmeldenummer: 07701855.4
(22) Anmeldetag: 02.02.2007
(51) Int. Cl.: A61F 6/14, A61K 9/00

(54) **RINGPESSAR IM UTERUS**
RING PESSARY IN THE UTERUS
PESSAIRE ANNULAIRE A PLACER DANS L'UTERUS

(30) Priorität: 28.02.2006 CH 318062006
(43) Veröffentlichungstag der Anmeldung: 12.11.2008
(73) Patentinhaber: Györffy, Gabor, 4104 Oberwil BL (CH)
(72) Erfinder: Györffy, Gabor, 4104 Oberwil BL (CH)
(74) Vertreter: Mak, Andras
(86) Internationale Anmeldenummer: PCT/CH2007/000055
(87) Internationale Veröffentlichungsnummer: WO 2007/098618

(56) Entgegenhaltungen:
- WO-A-95/00118
- US-A- 1 896 071
- US-A- 3 659 597
- US-A- 4 111 196
- US-A- 4 146 023
- US-A- 4 194 503

## Beschreibung

### Technisches Gebiet

Die Erfindung betrifft ein Verfahren und einen Intrauterinpessar (IUP) für die Schwangerschaftsverhütung und Einführvorrichtung für den Pessar. Im Einzelnen geht die Erfindung von einem Intrauterinpessar gemäß Oberbegriff von Anspruch 1 aus.

Für die Empfängnis oder Schwangerschaft verhürung kommen unterschiedliche Mittel und Methoden zur Anwendung. Obwohl die chemischen und hormonellen Wirkstoffe weit verbreitet und mit hoher Sicherheit verwendet werden können, gibt es Fälle wo die mechanischen Mittel und Methoden besser geeignet oder sogar ausschließlich verwendet werden können.

Zu den mechanischen Mitteln gehören die Kondome für die Männer sowie die intravaginalen und intrauterinen Mitteln (wie z. B. Diaphragma, Portiokappe, und Spirale) für die Frauen. Dabei sind die Spirale die billigsten und bequemsten Kontrazeptiven, da sie nach dem Einsetzen 2-3 Jahre verwendet werden können ohne, dass irgendeine andere Maßnahme für die Schwangerschaftsverhütung erforderlich wäre.

Eines der ersten Intrauterinpessare hatte die Form einer Spirale wodurch die IUP Implantate häufig als Spirale bezeichnet werden. Die Spirale hat den Vorteil, dass man sich nicht mehr täglich um die Verhütung kümmern muss. Wenn man die Pille aus medizinischen Gründen nicht nehmen kann oder aus persönlichen Gründen nicht nehmen will, ist die Spirale eine sehr gute Alternative.

Es sind verschiedene Formen von Spiralen bekannt. Die bekanntesten haben eine O-, Y oder T-Form. Die Spirale mit der O-Form werden auch als Ringspirale bezeichnet. WO95/00118 beschreibt eine extrakorporale Ringspirale, die bereits außerhalb des Körpers geschlossen wird und als ein geschlossener Ring in den Uterus eingesetzt wird. Solche extrakorporale Ringspirale können entweder unmittelbar nach der Geburt oder durch einen gyneakologischen Eingriff nach Erweiterung des Muttermunds unter Narkose eingeführt werden. Dies bedeutet eine starke Belastung des Körpers der Frau und eine Infektion kann auch nicht ausgeschlossen werden.

Eine bessere Lösung ergibt sich, wenn dir Ringspirale in langgestreckter Form in den Uterus eingeführt und erst nach der Einführung zu einem Ring geschlossen wird. Aus US 1,896,071; US 4,111,196 und US 4,194,503 sind Ringspirale bekannt, die mit Hilfe einer Einführungsvorrichtung in langgestreckter Form in den Uterus eingeführt und erst nach der Einführung zu einem Ring teilweise geschlossen werden. Die Abmessung der Ringform ergibt sich konstruktiv aus dem elastischen Material der Ringspirale und wird geringfügig auch durch die Gegenkraft der Uterus-Muskulatur beeinflusst. Diese Ringspirale sind zwar wesentlich einfacher implantierbar, haben jedoch den gemeinsamen Nachteil, dass bedingt durch die offenen Enden mit der Zeit eine Schenkelverlagerung eintritt, da durch die Kontraktion vom Cavum, Kräfte freigesetzt werden, die auf die Schenkel der Spirale wirken.
Die Schenkelverlagerung führt zu Druckstellen auf dem Endometrium. Der ständige Druck auf eine kleine Fläche fördert die Uterus-Kontraktionen noch zusätzlich was frühzeitig zu Beschwerden führt. Folglich wird eine solche Ringspirale entweder ausgestoßen oder wenn der offene Ring nicht selber herauskommt, soll es wegen den Beschwerden - wie z.B. Stechen, Schmerzen und Blutung - entfernt werden. Die Expulsionsrate der bekannten Ringspirale ist ebenfalls zu hoch (20-30%) um diese als sichere und langzeitige Verhütungsmittel einsetzen zu können.

Aus US 3,659,597 ist eine Ringspirale bekannt, die die mit Hilfe einer Einführungsvorrichtung in aufgerollter Form in den Uterus eingeführt wird. Nach der Einführung entspannt sich das elastische Material und öffnet sich zu einem Ring mit einer Abmessung die auch von der Abmessung der Cavum Uteri bestimmt wird. Dadurch ergibt sich der Vorteil, dass sich die Ringspirale konzentrisch positionieren kann innerhalb der Cavum Uteri. Da diese Ringspirale nicht geöffnet werden kann und auch nicht mit einem Faden verbunden ist, kann nur mit Hilfe einer Entnahmevorrichtung entfernt werden.

### Beschreibung der Erfindung

Die Aufgabe der Erfindung besteht deshalb darin, einen Intrauterinpessar der eingangs genannten Art zu Schaffen, der ohne besondere Maßnahmen einfach und schmerzlos (ohne Dilatation und Aneasthesie) in den Uterus eingeführt werden kann und sich dort optimal positioniert und bei Bedarf genauso einfach wieder entfernt werden kann. Eine weitere Aufgabe der Erfindung besteht darin, einen Intrauterinpessar anzugeben, der es ermöglicht, die Verletzungsgefahr und die Expulsionsrate wesentlich zu reduzieren.

Diese Aufgabe wird erfindungsgemäß durch einen Intrauterinpessar gelöst, der einen stabförmigen Grundkörper aus elastischem material besitzt, der beim Einlegen durch eine rohrförmige Einführungsvorrichtung geschoben, und dabei in eine im wesentlichen Kreisform gebogen werden kann, so dass die beiden Enden zusammengehalten werden. Dazu ist erfindungsgemäß vorgesehen, dass am einen Ende der stabförmigen Spirale ein Faden befestigt ist, das durch eine am anderen Ende der Spirale vorgesehene Durchführungsöffnung geführt ist, wobei am Faden in der Nähe des Befestigungspunktes ein Arretierungselement vorgesehen ist, und das dem Befestigungspunkt entgegengesetzte Ende des Fadens an der Einführungsvorrichtung befestigt ist.

Bei einer solchen Ausführung wird nicht nur eine einfache Einführung möglich, die nicht zu einem Zeitpunkt unmittelbar nach der Entbindung festgelegt ist, sondern in einem beliebigen Zeitpunkt ohne Vollnarkose und im wesentlichen schmerzlos durchgeführt werden kann. Durch die kontinuierliche Schleifenbildung und damit einer ständig im wesentlichen geschlossene Ringform kann die Gefahr eines Einhakens der Spitze oder sogar einer Perforation durch die Spitze vermieden werden und es ergibt sich eine optimale Positionierung innerhalb der Cavum Uteri und durch gleichmäßiges Aufliegen auf der Gebärmutterschleimhaut treten auch keine unerwünschte Nebenwirkungen oder Beschwerden auf.

Zur Lösung der Aufgabe wird auch ein Verfahren zur Einführung eines Intrauterinpessars vorgeschlagen, das insbesondere zur Einführung eines Intrauterinpessars gemäß der Erfindung geeignet ist. Es wird dabei ein stabförmiger Grundkörper mit zwei Enden aus elastischem material in langgestreckter Form in eine Einführvorrichtung mit einem Rohr und einem Ausstoßelement hineingesetzt und damit zusammen durch die Öffnung des Muttermundes in die Gebärmutter hineingeführt. Der Intrauterinpessar aus dem Rohr wird hinausbewegt und bei der Hinausbewegung wird eine Ringform des Pessars herbeigeführt. Erfindungsgemäβ ist vorgesehen, dass am ersten Ende des Grundkörpers ein erstes Ende eines Fadens befestigt ist, der Faden durch eine Öffnung am zweiten Ende des Grundkörpers hindurchgeführt wird und das zweite Ende des Fadens an der Einführvorrichtung befestigt wird, wobei die beiden Enden des Grundkörpers nach der Einführung miteinander fest verbunden werden.

Es wird weiterhin auch eine Vorrichtung zur Einführung eines Intrauterinpessars, vorgeschlagen, die insbesondere zur Einführung eines erfindungsgemäßen Intrauterinpessars geeignet ist. Eine solche Vorrichtung weist im allgemeinen ein Rohr und ein im Rohr längsverschiebbar angeordnetes Ausstoßelement auf, wobei erfindungsgemäß am Rohr ein Befestigungspunk für einen, an einem ersten Ende des Grundkörper des Intrauterinpessars befestigten Faden vorgesehen ist.

### Kurze Beschreibund der Zeichnung

Die weiteren Einzelheiten und Vorteile der Erfindung werden anhand der in der Zeichnung dargestellten Ausführungsbeispiele näher erläutert. Es zeigen:
Fig. 1 eine Einführungsvorrichtung mit einem Zahnrad und einer Zahnstange,
Fig. 2 eine Einführungsvorrichtung mit einem Flaschenzugsystem,
Fig. 3 eine Einführungsvorrichtung mit einem Abzug-Getriebe mit Federn,
Fig. 4 eine Einführungsvorrichtung mit einem Finger-Druck System,
Fig. 5 eine Einführungsvorrichtung mit einem handgetriebenem Hebelsystem,
Fig. 6 eine Einführungsvorrichtung mit einem handgetriebenem Kolbensystem,
Fig. 7 eine handgetriebene Einführungsvorrichtung mit Trokar und Hülse,
Fig. 8 die erfindungsgemäße Ringspirale im offenem Zustand,
Fig. 9 eine Fadenschneidevorrichtung für die Einführungsvorrichtung, und
Fig. 10 eine Einführungsvorrichtung mit der eingesetzten Spirale.

### Reschreibung der Ausführungsbeispiele

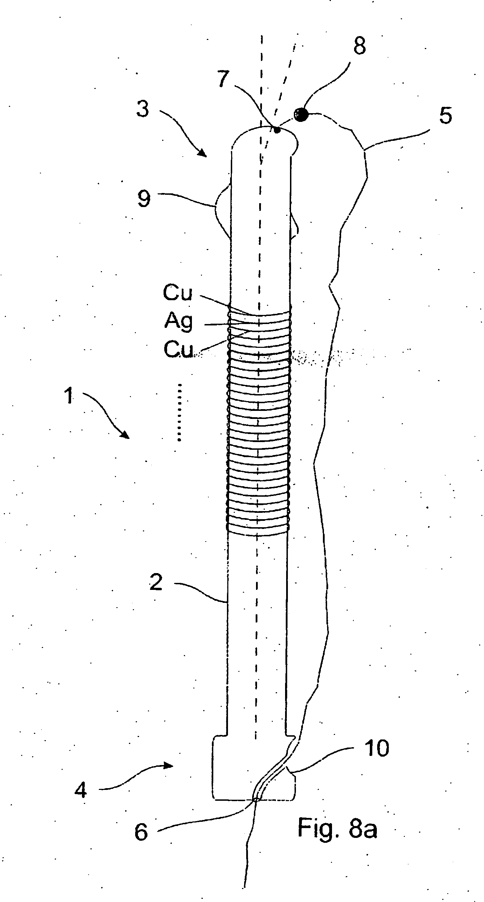

Der erfindungsgemäße Intrauterinpessar (Ringspirale) ist am besten in Fig. 8 erkennbar. Fig. 8 zeigt die Ringspirale 1 in der langgestreckten Form wie es vor der Einführung aussieht. Der Intrauterinpessar der Erfindung weist einen stabförmigen Grundkörper 2 aus elastischem material auf, der beim Einführen durch eine rohrfömige Einführmigsvorrichtung geschoben wird. Während der Einführung wird der stabförmige Grundkörper 2 in eine Kreisform gebogen, wobei die beiden Enden 3,4 zusammengehalten werden. Dazu ist am einen Ende 3 der stabförmigen Spirale ein Faden 5 befestigt, der durch eine am anderen Ende 4 der Spirale vorgesehene Durchführungsöffnung 6 geführt ist. Am Faden 5 in der Nähe des Befestigungspunktes 7 ist zusätzlich ein Arretierungselement 8 vorgesehen, und das dem Befestigungspunkt 7 entgegengesetzte Ende des Fadens 5 ist an der Einführungsvorrichtung befestigt.

Bei der Einführung eines Intrauterinpessars 1, wird der stabförmige Grundkörper 2 mit den zwei Enden 3, 4 aus elastischem material in langgestreckter Form in eine Einführvorrichtung mit einem Rohr und einem Aussteßelement hineingesetzt. Das Rohr mit der eingelegten Spirale wird durch die Öffnung des Muttermundes in die Gebärmutter hineingeführt, und anschließend wird der Intrauterinpessar aus dem Rohr hinausbewegt und bei der Hinausbewegung wird eine Ringform des Pessars herbeigerührt. Vor dem Einführen wird am ersten Ende 3 des Grundkörpers ein erstes Ende eines Fadens 5 befestigt. Der Faden wird mit dem freien Ende durch eine Öffnung 6 am zweiten Ende 4 des Grundkörpers 2 hindurchgeführt und das noch freie zweite Ende des Fadens wird an der Einführvorrichtung befestigt. Die beiden Enden 3, 4 des Grundkörpers 2 werden nach der Einführung miteinander fest verbunden.

Zur Befestigung der beiden Enden 3, 4 dient das Arretierungsmittel. Das Arretierungselement kann als ein Knopfelement 8 ausgebildet sein, wobei die größte Abmessung des Knopfelementes geringfügig größer ist als die größte Abmessung der Durchführungsöffnung 6. Der Abstand vom Knopfelement 8 zum Befestigungspunkt 7 wird vorteilhafterweise so gewählt, dass im geschlossenen Zustand der Spirale die beiden Enden 3,4 der Spirale aneinander zum Liegen kommen und in dieser Lage gehalten werden. Das Knopfelement verhindert dabei eine selbstständige Öffnung der Spirale.

Das eine Ende der Spirale 3 ist als ein Kopf mit einer abgerundeten Spitze und einem in einer Richtung ausweitendem Bauch 9 ausgebildet. Der Kopf 3 zeigt eine Neigung in einem kleinen Winkel wegen Einführungserleichterung. Bei evtl. Formveränderungen im Utero mit eventuellen Zusatz-Drehbewegungen kann dann ein solcher Kopf besser ausweichen. Das andere Ende ist als Fuß 4. mit einer Einbuchtung 10 ausgebildet. Im geschlossenen, im wesentlichen kreisförmigen Zustand wird die abgerundete Spitze durch die Einbuchtung aufgenommen. Die Durchführungsöffnung 6 ist im Fuß 4 schräg ausgebildet, wobei der Faden 5 im Seitenbereich hineingeführt und im Mittelbereich hinausgeführt ist. Die Spirale 1 besteht in einer vorteilhaften Ausgestaltung der Erfindung aus einem Plastikwerkstoff, und an der Spirale sind in Längsrichtung abwechselnd zwei unterschiedliche Metallteile angebracht. In der dargestellten Ausführung sind diese Metalle Kupfer (Cu) und Silber (Ag). Bei der Spirale ist es wichtig , dass zwei verschiedene Metalle abwechselnd angebracht werden damit das Volta Prinzip zu Gunsten des Pearl Index ausgenützt werden kann. (.."wo zwei verschieden Metalle sich berühren, entsteht eine Mikrospannung"). Auβerdem ist es auch möglich, die Spirale mit einem Wirkstoff, gegebenenfalls mit einer entsprechend ausgewählten Hormone zu ergänzen. Die Verwendung von Metall und/oder Hormone zur Erhöhung der Wirkung der Spiralen ist an für sich bekannt.

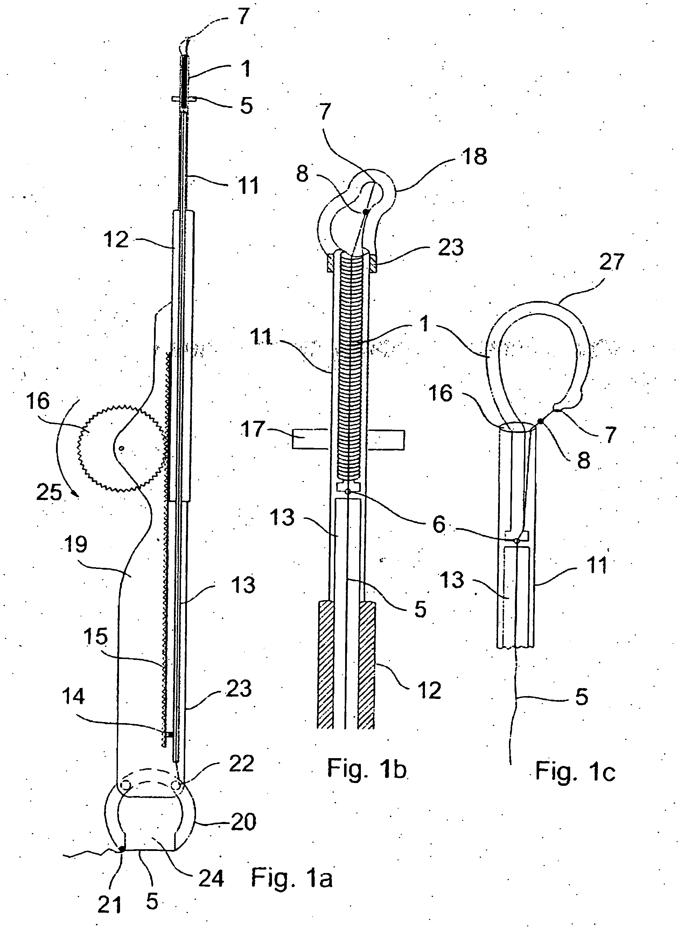

Um eine Verletzung im Uterus durch Perforation zu vermeiden ist es besonders wichtig, dass der stabförmige Pessar in einer Schleifen- oder Kreisform in den Uterus eingeführt wird. Bei der erfindungsgemäßen Ringspirale wird die Kreisform oder kreisähnliche Form kontinuierlich beim Einschieben dadurch erzielt, dass das distale Ende 3 der Spirale mittels Einzugsfaden 5 festgehalten wird. Dadurch wird das erste oder distale Ende 3 der Spirale immer in der Nähe der Ausgangsöffnung 16 des Einführungsrohrs 11 gehalten, wie es in Fig. 1c am besten zu sehen ist. Wenn der Grundkörper vollständig aus dem Rohr geschoben ist, wird das Arretierungselement oder Knopfelement 8 durch die schräge Durchführungsöffnung 6 gezogen. In diesem geschlossenen Zustand liegen die beiden Enden 3, 4 aneinander auf und das Knopfelement - da es geringfügig größer ist als die Durchführungsöffnung-verhindert eine zufällige Öffnung der Spirale.

Die erfindungsgemäße Ringspirale kann mit einer von mehreren Einführungsvorrichtungen in den Uterus eingeführt werden. Einige Beispiele sind in den Figuren 1-7 und 8-10 gezeigt. Die Einführungsvorrichtungen haben die gemeinsame Funktion, den Intrauterinpessar in den Uterus zu fördern und dabei möglichst eine Infektion zu vermeiden. Das Funktionspnnzip der Einführyorrichtungen ist immer dasselbe: die Spirale wird aus dem Einführungsrohr mittels Kolben ausgestoßen. Bei einem Trokar-Hülse System (wie in WO 01/76516 beschrieben) welches nach dem Prinzip der Laparoskopie funktioniert, sind auch weitere Komponenten erforderlich. Das eigentliche Einführungsrohr ergibt sich hier aus einer zusammengesetzten Hülse und Vertängerungsrohr. Der Kolben wird mittels Getriebe oder per Hand betätigt. Die unterschiedlichen Betätigungsmöglichkeiten werden in der nachstehenden Beschreibung näher erläutert.

Fig. 1 zeigt ein Zahnrad und Zahnstangen System, wobei ein Kolben 13 mit einer Zahnstange 15 fest verbunden ist und die Zahnstange mit einem Zahnrad 16 in Längsrichtung verschoben werden kann. Durch diese Ausführungsvariante wird eine Drehbewegung vom Zahnrad 16 in eine Langsbewegung der Zahnstange 15 und des damit verbundenen Kolbens 13 umgewandelt. Das Einführungsrohr besteht in diesem Beispiel aus zwei Abschnitten, aus einem elastischen Rohrabschnitt 11 und einem verstarkten Rohrabschnitt 12. Der verstärkte Rohrabschnitt ist mit einem Griff 19 versehen, der das Zahnrad 16 verdrehbar lagert und endseitig mit einem Fadenführer 20 verbunden ist. Am elastischen Rohrabschnitt 11 ist weiterhin eine Arretierung 17 vorgesehen. Mit dieser Arretierung kann die Länge eingestellt werden, die es bestimmt, wie weit das Einführungsrohr in den Uterus eindringt. Bei der Erfindung wird nur ein Teil des Systems eingeführt, nicht wie gewöhnlich bis zum Fundus Uteri, sondern nur bis zum Ende des Canalis Uteri.

Vor dem Einsetzen wird der Intrauterinpessar 1 in den elastischen Abschnitt des Einführungsrohrs 11 gelegt und zwar in einer langgestreckten Form. Die Spitze 3 der Spirale ragt über das Rohrende 16 und ist mit einem Hütchen (Kondom) 18 abgedeckt, um die eventuell vom Muttermund verschleppten Bakterien mit der ganzen Vorrichtung entfernen zu können, da das Hütchen 18 an dem Rohr 11 fixiert ist. Der Kolben 13 liegt zwischen dem proximalen Ende 4 der Spirale 1 und dem Fadenführer 20. Der Kolben 13 ist ein dünnes starres Plastikrohr (oder anderes Material) auf jeden Fall muss es starr genug sein. So dass der Faden 5 von der Spitze 7 der Spirale durch das Loch 6 der Spirale verläuft, neben dem Kolben 13 durch den Fadenführer 20 bis zum Befestigungspunkt 21. Der Kolben 13 ist mit der Zahnstange 15 durch ein Verbindungsstück 14 verbunden. Wenn die Zahnstange 15. durch Drehung des Zahnrades 16 in Pfeilrichtung 25, nach vorne bewegt wird, bewegt sich der Kolbens 13 auch nach vom. Der Kolbens 13 ist vom Ende des Rohres bis zum Fadenführer 20 mit einer Plastikplatte 23 abgedeckt, so dass der Kolben, auch wenn er sehr dünn ist nicht verbiegen kann, und die Kraft nach vorne wirkt. Wenn wir das Zahnrad 16 mit dem Daumen in Pfeilrichtung 25 nach hinten drehen, bewegt sich die Zahnstange 15 nach vorne und gleichzeitig drückt den Kolben 13 nach vorne. Der Kolben 13 drückt die Spirale 1 aus dem Rohr 11 während der Faden 5 im Befestigungspunkt 7 und 21 fixiert bleibt. Da der Faden 5 der Spirale an beiden Enden fixiert ist, beugt sich die Spitze der Spirale 1 sofort und es entsteht allmählich eine immer größer werdende Schleife 24 dadurch, dass die beiden Enden der Spirale sich nähern, nährend die Spirale 1 aus dem Einführungsrohr 11 hinausgetrieben wird. Damit ergibt sich der Vorteil, dass durch eine primäre Schleifenbildung die Spitze der Spirale nicht in die Uteruswand oder eine Tubenecke einhaken und demzufolge auch keine Perforation verursachen kann. Das Hütchen 18 wird durch die Kraft der Schleife schon zu Beginn zum platzen gebracht.

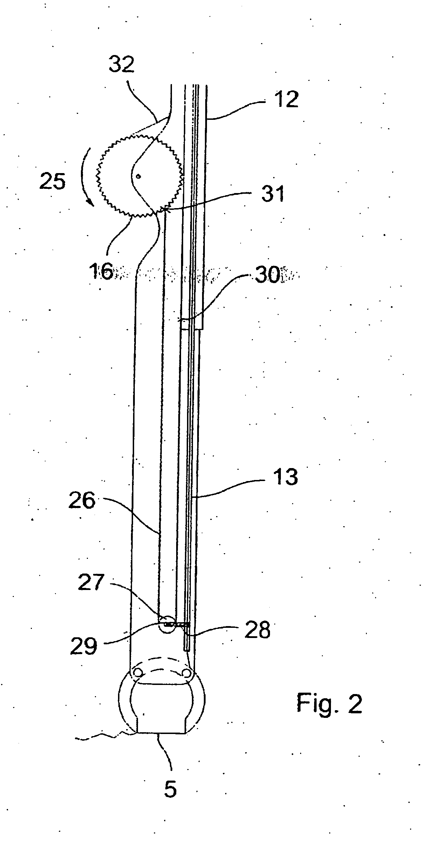

Fig. 2 zeigt nur das Getriebe mit einem Flaschenzug System. Die mit den Elementen von Fig. 1 identischen Elemente sind mit gleichen Bezugszeichen versehen. Bei dieser Ausführung ist der verstärkte Rohrabschnitt 12 mit einem Seil 26 verbunden, das über ein frei laufendes, am Kolben befestigtes Rad 27 geführt ist und mit seinem Ende am Zahnrad 16 befestigt ist. Das frei laufende Flaschenzug-Rad 27 ist durch ein Verbindungsstück 28 mit dem Kolben 13 fest verbunden. Der eigentliche Unterschied zum Fig. 1 besteht darin, dass die Zahnstange durch den Flaschenzug Rad 27 versetzt wurde. Das Seil 26, das um das Rad 27 gewickelt ist, ist einerseits im Punkt 30 fixiert, das andere Ende im Punkt 31 befestigt. Durch das Drehen des Zahnrades 16 bewegt sich der Kolben 13 nach vorne, weil das Flaschenzug-Rad 27 am Kolben durch ein Verbindungsstück mit zwei Schenkeln 28 und einer Achse 29 fixiert ist. Durch diese Ausführung ergibt sich der Vorteil, dass dieses Systems die halbe Kraft beim Einlegen der Spirale und weniger Material benötigt. Hier ist gegenüber dem Zahnstangen-System eine Arretierung 32 notwendig, damit ein Zurückschnellen der Spirale und des Kolbens 13 verhindert wird. Dies wird beim Zahnrad-Zahnstangen System durch die Reibung zwischen den Zähnen des Zahnrades und der Zahnstange verhindert. Die Arretierung 32 ist eine dünne Plastikplatte, die im entsprechenden Winkel angelegt ist und das Zurückdrehen des Zahnrades 16 verhindert. Folglich ist die Bewegung nur in einer Richtung 25 ermöglicht. Der Aufbau der Vorrichtung ist im übrigen wie beim Zahnstangen-System. Durch Drehung des Zahnrades 16 in Pfeilrichtung 25 wird das Seil 26 auf das Zahnrad 16 aufgerollt und dabei der Kolben 13 nach oben bewegt die Spirale aus dem Rohr hinausgedrückt.

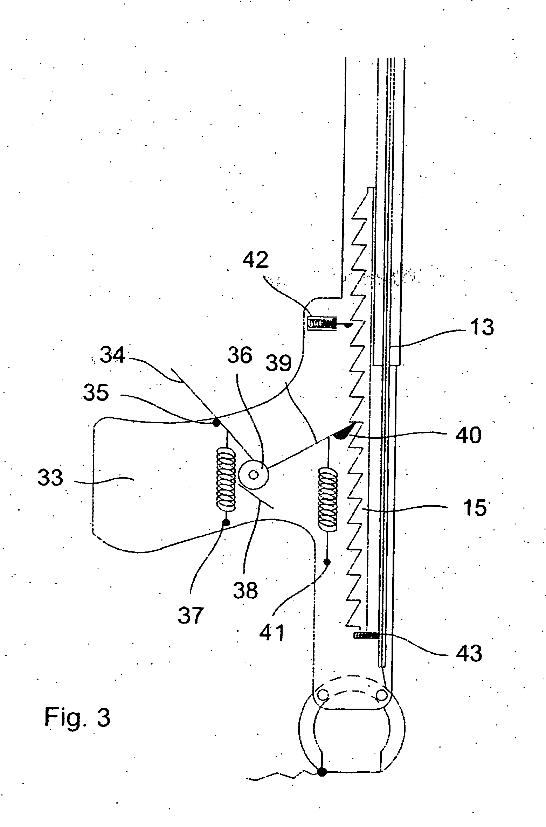

Fig. 3 zeigt ein Abzug-Getriebe mit drei Feldern, wobei der Kolben 13 wie bei Fig. 1, mit einer Zahnstange 15 verbunden ist. Das Zahnrad von Fig. 1 und 2 ist jedoch durch einen Abzug-Mechanismus mit einem Gelenksystem ersetzt. Der Kolben 13 wird hier wie bei Fig. 1 durch die Zahnstange 15 mitgenommen, welche durch einen Abzug-Mechanismus mit einem Gelenksystem vorwärts getrieben wird. Der Griff 33 muss einen Revolver- oder Pistolen ähnliche Form haben. Der Abzug 34 ist um eine Achse 35 drehbar gesichert. Das Gelenk 36 ist beweglich. Der Abzug 34 wird in die ursprüngliche Position durch die Zugfeder 37 zurückgebracht. Ein Anschlag 38 verhindert den übermäßigen Rückzug des Gelenkes 36. Der Schenkel 39 bleibt immer auf der Zahnstange 15. Die Spitze 40 des Schenkels und die Zähne der Zahnstange 15 sind so ausgebildet, dass sie eine Vorwärtsaber keine Rückwärts-Bewegung erlauben. Eine zweite Zugfeder 41 hält den Schenkel 39 auf der Zahnstange 15. Eine Arretierung 42 mit einer Spitze ist so ausgebildet, dass ein zurückschnellen der Zahnstange 15 verhindert wird. Die Druckfeder in der Arretierung 42 drückt auf die Zahnstange 15, aber die Spitze ist so ausgebildet, dass es Bewegungen nur in eine Richtung erlaubt. Bei der Einführung wird der Abzug 34 mehrere Male gezogen und losgelassen. Beim Ziehen des Abzugs wird die Spitze 40 des Schenkels gegen einen Zahn der Zahnstange 15 gedrückt und dadurch die Zahnstange und der damit verbundene Kolben 13 vorwärts bewegt so lange, bis die Spitze 40 den Kontakt mit den Zahnstange verliert. Beim Loslassen des Abzugs 34 zieht die Zugfeder 41 den Schenkel 39 mit Spitze 40 zurück in die ursprüngliche Position. Der Anschlag 38 begrenzt dabei die Bewegung des Gelenks 36 und des damit verbundenen Abzugs 34 und Schenkels 39. Die Zahnstange 15 ist mit dem Kolben 13 verbunden durch ein Verbindungsstück 43, wodurch sich der Kolben 13 mit jedem Abzug ein keines Stück nach vorwärts bewegt. Die Spirale wird dabei durch die intermittierende Kolbenbewegung hinausgepresst.

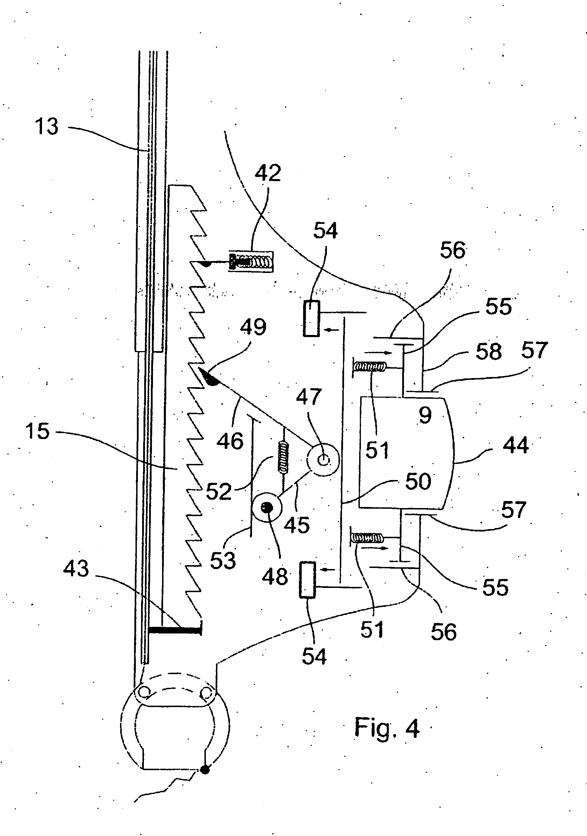

Fig. 4 zeigt ein Finger-Druck System, das ähnlich wie das System mit dem Abzug funktioniert. Die Zahnstange 15 wird hier ebenfalls durch ein Gelenkssystems vorwärts getrieben der Abzug ist jedoch mit einem Druckknopf 44 ersetzt. Die Vorwärtsbewegung wird durch Drücken und loslassen des Druckknopfes gewährleistet.

Bei diesem Getriebesystem ist der Kolben 13, wie in Fig. 1 und 3, mit einer Zahnstange 15 verbunden. Zwischen den Zähnen der Zahnstange greifen ein Gelenksystem für die Vorwärtsbewegung und ein Arretierungselement 42 für die Verhinderung der Zurüekbewegung ein. Das Gelenksystem besteht aus einem kürzeren Schenkel 45 und einem längeren Schenkel 46, die über ein Gelenk 47 miteinander verbunden sind. Der Schenkel 45 ist um eine feste Achse 48 drehbar gelagert und eine Spitze 49 vom Schenkel 46 greift zwischen die Zähne der Zahnstange 15. Das Gelenksystem wird mit einem Druckknopf-Mechanismus betätigt, der aus einem Druckknopf 44 und einer Druckplatte 50 besteht.

Die Zahnstange 15 bewegt den Kolben 13 wie bei Fig.1, durch den Druck auf den Druckknopf 44 und durch die Druckplatte 50 wird das Gelenksystem zusammengedrückt. Der auf der Zahnstange liegende Schenkel 46 versetzt die Zahnstange 15 in Vorwärtsbewegung. Die Zahnstange 15 wird nach dieser Bewegung von einer Arretierung 42 festgehalten, ähnlich wie bei dem Abzug System, folglich nur eine Vorwärtsbewegung ist ermöglicht, die Rückwärtsbewegung ist gesperrt.

Wähnend des Loslassens des Knopfes aktivieren sich die drei Federn. Davon zwei Federn 51 drücken den Druckknopf 44 zurück, während die dritte Feder 52 zieht das Gelenksystem in die ursprüngliche Position. Ein Anschlagelement 53 verhindert ein übermäßiges Zurückziehen des Gelenksystems. Zwei weitere Anschlagelemente 54 verhindern ein übermäßiges Zusammendrücken des Gelenksystems. Die Stabilisierungsflügel 55 bewegen sich zwischen zwei Schienen 56 und der Druckknopf ist ebenfalls zwischen zwei Schienen 57 geführt um eine bessere Bewegung des Druckknopfes zu gewährleisten und ein Einklemmen zu verhindern. Die ganze Konstruktion befindet sich im Griffgehäuse 58.

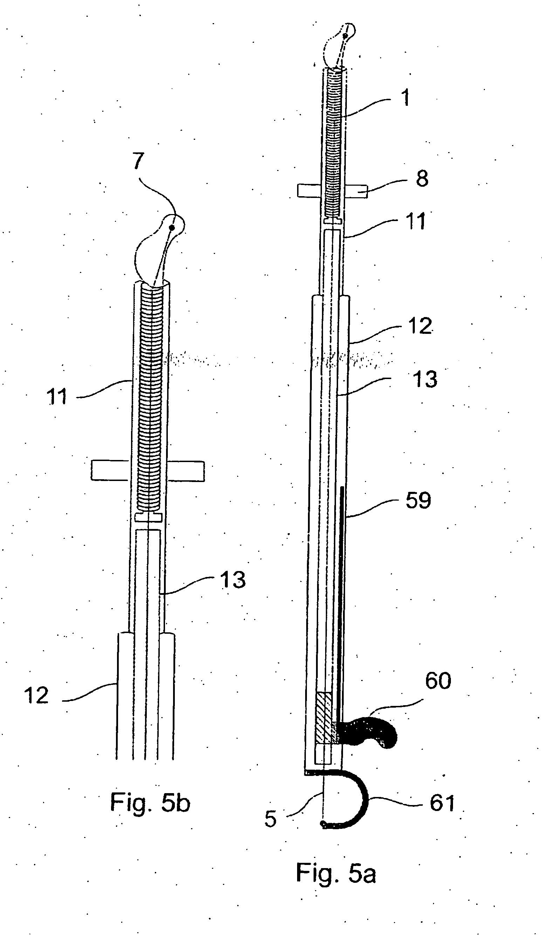

Fig. 5a zeigt ein Handgetriebenes System mit einem Hebel. Fig. 5b zeigt einen Ausschnitt von diesem System in einem vergrößerten Meßstab. Das einfachste und wahrscheinlich billigste System besteht aus zwei Rohrabschnitten, aus einem starren Rohrabschnitt 12 und einem weichen, elastischen Rohrabschnitt 11. Der stange Rohrabschnitt 12 besitzt einen Schlitz 59. Die Länge des Schlitzes 59 richtet sich nach der Länge der Spirale, so dass sie die Knopf-Loch-Schtießung erlaubt. Der Kolben 13 wird durch den am Kolben fixierten Hebel 60 betätigt. Ein Halbring 61 dient für die Spannung des Fadens 5. Die Spirale 1 wird hier direkt mit dem handbetätigten Hebel 60 aus dem Einfuhrungsrohr gedrückt und der Faden wird zum Schluss mit einer Schere durchgeschnitten.

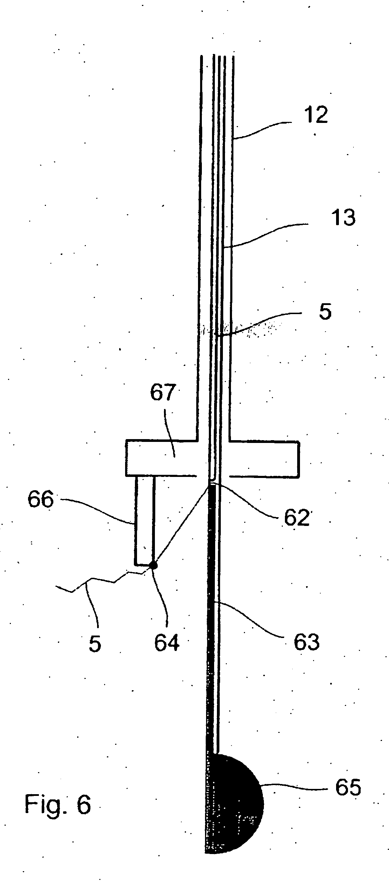

Fig. 6 zeigt ebenfalls ein Handbetriebenes System, jedoch mit einem Kolben, der den Hebel von Fig. 5 ersetzt. Dieses System besteht aus einem Rohr 12 mit einem Kolben 13. Der Kolben 13 ist mit einer Kolbenstange 63 verbunden, der am anderen Ende mit einem Griff 65 versehen ist. Der Faden 5 der Spirale wird seitwärts durch den Kolbens 13 und das Rohr 12 hinausgeführt und im Punkt 64 an einem Stift 66 befestigt, der mit einem Anschlag 67 verbunden ist. Das andere Ende des Fadens ist, wie bei allen anderen Systemen, am Kopf der Spirale fixiert.

Durch das Hineinschieben des Kolbens 13 wird der Kolben gegen die Spirale gedrückt und die Spirale wird dadurch hinausbewegt. Von dem Seitenloch 62 bis zum Griff 65. muss die Kolbenstange 63 geschlitzt sein oder viel dünner als das Rohr 12 sein, damit der Faden 5 zwischen dem Rohr 12 und dem Kolben 13 nicht bremsen kann, oder nicht einklemmen kann während der Kolben hineingeschoben wird Auch hier wird die Spirale direkt mit dem handbetätigten Kolben aus dem Einführungsrohr gedrückt und der Faden wird zum Schluss mit einer Schere durchgeschnitten.

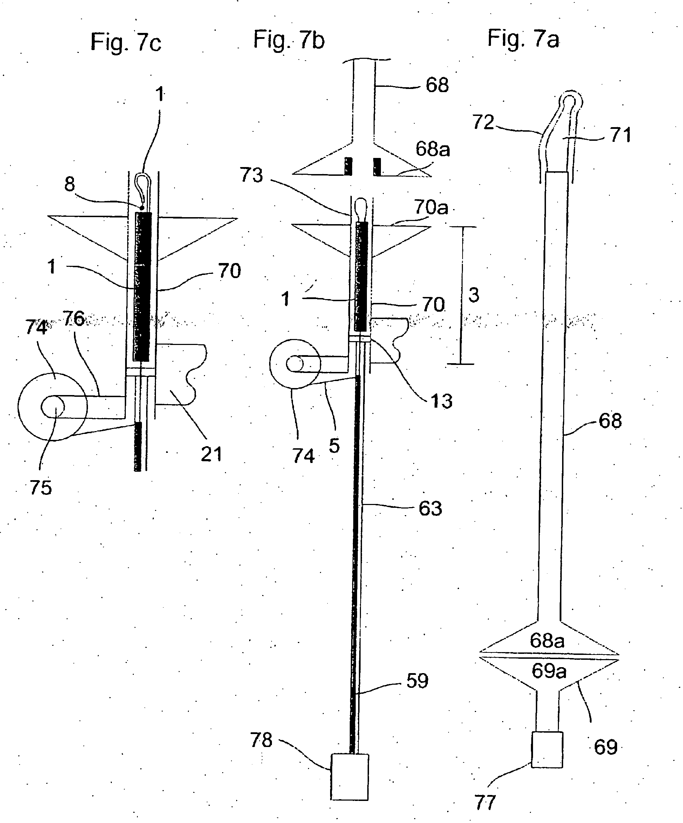

Fig. 7 zeigt ein Handgetriebenes System mit Trokar und Hülse wie es aus WO 01/76516 bekannt ist. Das Instrument besteht aus einer Hülse 68, einem Trokar 69 und einem Verlängerungsrohr 70. In Fig. 7a ist der Trokar 69 ist in die Hülse 68 hineingeschoben, wobei die Flächen 68a und 69a aneinander aufliegen. Die Spitze 71 des Trokars ist ähnlich gestaltet wie die Spitze der Spirale. Die Spitze 71 des Trokars wird mit einem Kondom 72 abgedeckt, dessen Rand frei ist und am Rohrende der Hülse 68 befestigt ist. Im Verlängerungsrohr 70 befindet sich die Spirale 1 und ein Teil des Kolbens 13 (Fig. 7b). Fig. 7c zeigt einen Ausschnitt von Fig. 7b im vergrößerten Maßstab.

Der Trokar 69 und die Hülse 68 werden gemeinsam in den Uterus bis zum Ende der Cervikal Kanal hinengeschoben (Fig. 7a). Der Trokar 69 wird mit dem Griff 77 entfernt während die Hülse 68 sitzen bleibt und festgehalten wird. Das Verlängcnmgsrohr 70 mit der Spirale 1 und dem Kolben 13 in situ wird an die Hülse 68 adaptiert, wobei die Flächen 68a und 70a einander angepasst sind. Ein Stutzen 73 des Verlängerungsrohres 70 dient für einen festen Anschluss, wie es in Fig. 7b am zu sehen ist. An der Spitze der Spirale 1 ist hier auch ein Faden 5 mit einem Knopf 8 befestigt. Das andere Ende von Faden 5 ist durch die Spirale und den Kolben durchgeführt und an unteren Ende des Verlängerungsrohrs 70 ausgeführt und in einem Punkt an der Vorrichtung befestigt. Die Länge des Fadens ist so bestimmt, dass die beiden fixierten Enden des Fadens 5 zu einer vollständigen Schließung der Spirale beitragen. Die überstehende Länge des Fadens ist zunächst auf ein am Verlängerungsrohrs 70 montierten Rad 74 aufgewickelt. Das Rad 74 ist um eine Achse 75 drehbar gelagert und durch Schenkel 76 mit dem Verlängerungsrohr 70 verbunden.

Bei dem Hineinschieben des Kolbens 13 mit dem Griff 78 rollt nur so viel Faden 5 ab wie benötigt wird. Selbstverständlich muss der Kolben 13 geschlitzt oder dünner sein als das Verlängerungsrohrs 70, damit das Einklemmen des Fadens verhindert wird. Auch hier wird die Spirale direkt mit dem handbetätigten Kolben aus dem Einführungsrohr gedrückt und der Faden wird zum Schluss mit einer Schere durchgeschnitten. Das Hülse-Trokar System ähnelt sich dem in der Gynäkologie bekannten System für die Laparoskopie.

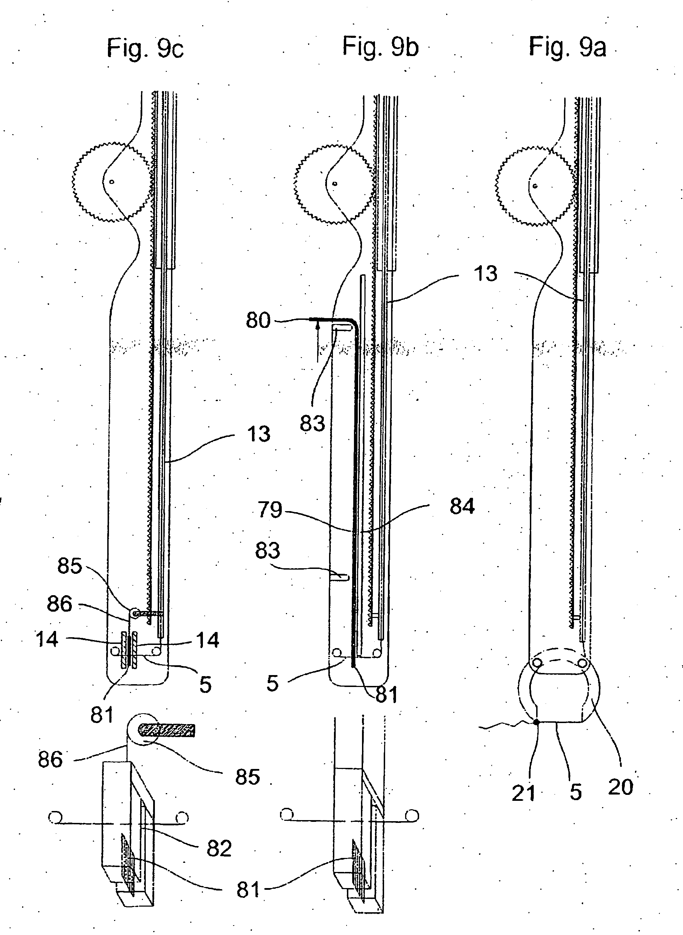

Fig. 9 zeigt unterschiedliche Fadenschneide-Systeme, die bei der Einführungsvorrichtung verwendet werden können. In der Zeichnung sind drei verschieden Fadenschneide-Systeme in Kombination mit dem Zahnrag-Zahnstangen System von Fig. 1 gezeigt: mit einer Schere, mit einem Hebel, mit einem auf einem Rad aufgewickelten Faden. Bei dem ersten System, wie es in Fig. 9a dargestellt ist, wird der durch den Kolben 13 verlaufende Faden 5 auf einer halbringförmigen Fadenführung 20 in einem Befestigungspunkt 21 fixiert, wo genügend Platz für eine Schere freigelassen ist.

Das zweite System (Fig. 9b) beinhaltet eine kleinen Stange 79 die mit einem Hebel 80, auf dessen anderen Ende sich ein Messer 81 befindet. Vor dem Messer 81 gibt es einen Spalt 82 hier läuft der Faden 5 durch. Nach der Einführung und Zusammenschließen der Enden der Spirale wird der Hebel 80 in eine Schneidposition gedrückt. Ein Hinausfallen der Stange 79 wird durch die Halterungen 83 verhindert. Eine Schiene 84 gewährleistet das reibungslose Verschieben der Stange 79 mit dem Messer 81.

Das dritte System (Fig. 9c) ist mit einem zusätzlichen Rad 85 versehen. Wenn der Kolben 13 bis zum Anschlag eingeführt wird, das Messer 81 mit einem Seil 86 in die Schneidposition bewegt, nach dem gleichen Prinzip wie bei System 2. Dadurch wird der Faden 5 automatisch abgeschnitten, da Fadenlänge auf den Abstand eingestellt ist. Bei dem Flaschenzug-System wird das dritte Schneide-System an das Flaschenzugsrad gebunden. Dabei sind untereinander zwei Räder angeordnet. Eine ist der Flaschenzugsrad und andere ist das Rad wo der Faden aufgerollt ist welche das Messer zichen wird erst dann wenn der Ring schon geschlossen ist da die Fadenlänge ebenfalls berechnet wurde. Die zwei Räder sind miteinander und mit dem Kolben verbunden.

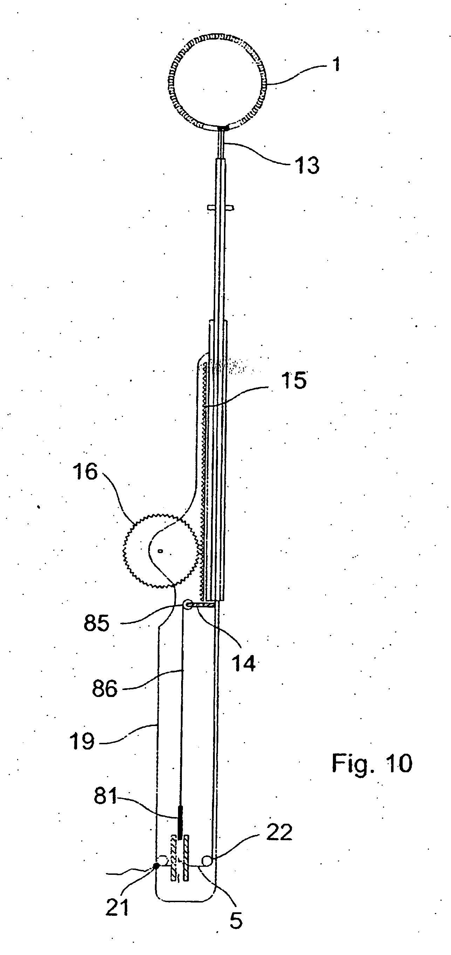

Fig. 10 zeigt ein Zahnrad-Zahnstangen System, wie im Zusammenhang mit Fig. 1 bereits ausführlich beschrieben wurde, mit einem Fadenschneide-System gemäß Fig. 9c und einer vollständig geschlossenen Ringspirale 1. Der Kolben 13 ist mit einer Zahnstange 15 über eine Verbindungsstück 14 fest verbunden. Die Zahnstange 15 wird durch Drehen des Rades 16 vorwärts getrieben. Der Faden 5 der Spirale ist an der Spitze der Spirale und an einem Befestigungspunkt 21 fixiert und um Stift 22 umgelenkt. Beim Hinausbewegen der Spirale wird ein Messer 81 das mittels Seit 86 von einem am Verbindungsstück 14 befestigtes Rad hineingezogen. Nach der vollständigen Schiessung der Spirale schneidet das Messer den Faden automatisch durch. Wie in Fig. 10 gezeigt, hat das Fadenschneide-System den Faden 5 der Spirale bereits durchgeschnitten, und der Kolben 13 ragt über den Einführungsrohr hinaus.

## Patentansprüche

1. . Intrauterinpessar (1) mit einem stabförmigen Grundkörper (2) aus elastischem Material, der mit zwei unterschiedlichen Metallteilen abwechselnd (Cu, Ag) oder mit einem Hormon versehen ist, beim Einführen durch eine rohrförmige Einführungsvorrichtung geschoben und dabei in eine im wesentlichen Kreisform gebogen werden kann, so dass die beiden Enden (3,4) des Grundkörpers zusammengehalten werden, **dadurch gekennzeichnet, dass** an einem Ende (3) des stabförmigen Grundkörpers an einem Befestigungspunkt (7) ein Faden (5) befestigt ist, der durch eine am anderen Ende (4) des Grundkörpers (2) vorgesehene Durchführungsöfnung (6) geführt werden kann, wobei am Faden (5) in der Nähe des Befestigungspunktes (7) ein Arretierungselement (8) vorgesehen ist, und das dem Befestigungspunkt (7) entgegengesetzte Ende (21) des Fadens an der Einführungsvorrichtung befestigbar ist.

2. . Intrauterinpessar gemäß Anspruch 1, wobei das Arretierungselement als Knopfelement (8) ausgebildet ist, wobei die größte Abmessung des Knopfelementes geringfügig größer ist als die größte Abmessung der Durchführungsöffnung (6).

3. . Intrauterinpessar gemäß Anspruch 1 oder 2, wobei der Abstand vom Knopfelement (8) zum Befestigungspunkt (7) so gewählt wird, dass im geschlossenen Zustand des Grundkörpers (2) die beiden Enden (3, 4) des Grundkörpers aneinander zum Liegen kommen und in dieser Lage gehalten werden.

4. . Intrauterinpessar gemäß Anspruch 1, wobei das eine Ende des Grundkörpers als ein Kopf (3) mit einer abgerundeten Spitze und einem in einer Richtung ausweitenden Bauch (9) ausgebildet ist und das andere Ende als ein Fuß (4) mit einer Einbuchtung (10) ausgebildet ist, wobei die abgerundete Spitze durch die Einbuchtung (10) aufgenommen wird.

5. . Intrauterinpessar gemäß Anspruch 4, wobei die Durchführungsöffnung (6) den Fuß (4) schräg durchstößt.

## Claims

1. Intrauterinpessar (1) with a rod-shaped main body (2) made from elastic material, which is provided in alternation with two different metal components (Cu, Ag) or with a hormone, and when being pushed during insertion through a tubular insertion device, it can be bent into a substantially circular shape so that the two ends (3, 4) of the main body are held together, **characterized in that** at one end (3) of the rod-shaped main body, at a point of attachment (7), a yarn (5) is fixed and can be lead through a feedthrough hole (6) provided at the other end (4) of the main body (2), wherein the yam (5) near the point of attachment (7) is provided with a locking element (8) and an end (21) of the yam opposite to the attachment point (7) may be attached to the insertion device.

2. Intrauterinpessar according to claim 1, wherein the locking element is formed as a button element (8), and the largest dimension of the locking element is slightly larger than the largest dimension of the feedthrough hole (6).

3. Intrauterinpessar according to claim 1 or 2, wherein the distance from the button element (8) to the point of attachment (7) is selected such that in the closed state of the main body (2) the two ends (3, 4) of the main body will be connected and held in this position.

4. Intrauterinpessar according to claim 1, wherein one end of the main body is formed as a head (3) with a rounded tip and a widening belly (9) extending in one direction and the other end if formed as a foot (4) with a recess (10), wherein the rounded tip is received by the recess (10).

5. Intrauterinpessar according to claim 4, wherein the feedthrough hole (6) intersects the foot (4) diagonally/in an oblique angle.

## Revendications

1. Intrauterinpessar (1) avec un corps principal en forme d'un bâton (2) fabriqué d'une matière élastique, qui, équipé avec deux métaux différents en alternance (Cu, Ag) ou avec une hormone, lors de l'insertion peut être poussé à travers un dispositif d'insertion tubulaire, et ainsi peut être plié en une forme essentiellement circulaire de sorte que les deux extrémités (3, 4) du bâton principal sont maintenues ensemble, **caractérisé en ce qu'** à une extrémité (3) du corps en forme d'un bâton, à un point de fixation (7), un fil (5) est fixé, et qui peut être enfilé à travers l'ouverture (6) qui s'est placée à l'autre extrémité (4) du corps de base (2), et le fil (5) près du point de fixation (7) est fourni avec un élément de verrouillage (8) et l'extrémité du fil opposée (21) au point de fixation (7) peut être reliée à l'appareil d'insertion.

2. Intrauterinpessar selon la revendication 1, dans lequel l'élément de verrouillage est formé comme un élément bouton (8), où la plus grande dimension de l'élément de fixation est légèrement plus grande que la plus grande dimension de l'ouverture du conduit (6).

3. Intrauterinpessar selon la revendication 1 ou 2, dans lequel la distance menant de l'élément bouton (8) jusqu'au point de fixation (7) est choisie de telle sorte qu'en état fermé du corps (2) les deux extrémités (3, 4) du corps principal se raccordent et sont maintenues dans cette position.

4. Intrauterinpessar selon la revendication 1, dans lequel l'une des extrémités du corps est formée comme une tête (3) avec un bout arrondi et un élargissement dans un sens comme un ventre (9) lorsque l'autre extrémité est formée comme un pied (4) avec un évidement (10), dans laquelle l'extrémité arrondie est reçue par le évidement (10).

5. Intrauterinpessar selon la revendication 4, dans lequel l'ouverture du conduit (6) passe obliquement à travers le pied (4).
